# EUROPEAN PATENT APPLICATION

(11) **EP 4 595 877 A1**
(43) Date of publication of application: **06.08.2025**
(21) Application number: 25153010.1
(22) Date of filing: 21.01.2025
(51) Int. Cl.: A61B 5/00, A61H 31/00

(54) **INFANT CARE SYSTEM AND METHOD**

(30) Priority: 05.02.2024 US 202418433064
(71) Applicant: GE Precision Healthcare LLC, Waukesha, WI 53188 (US)
(72) Inventor: BECKER, Karen P., Wauwatosa, 53226 (US); FALK, Steven M., Wauwatosa, 53226 (US)
(74) Representative: Fennell, Gareth Charles

(57) **Abstract**

A method of monitoring a neonate includes sensing a force applied to an infant support platform supporting a neonate, each load cell is configured to receive at least a portion of an applied force on the infant support platform while the neonate is on the infant support platform and to generate a load signal indicative of the portion of the applied force received. The load signal over time is analyzed during performance of a chest compression on the neonate, including determining a slope value, an impulse value, a jerk value, or a combination thereof for the chest compression based on the load signal and comparing the slope value to a slope threshold, the impulse value to an impulse threshold, and/or the jerk value to a jerk threshold to generate a comparison result for the chest compression. A user interface device is controlled to generate a care instruction based on the comparison result.

## Description

### BACKGROUND

The present disclosure generally relates to neonatal care, and specifically to a system and method for monitoring and assisting in chest compressions performed during resuscitation of neonatal patients.

Cardiac compression may be performed on a newborn in need of resuscitation, such as immediately after birth in labor and delivery. The optimal technique for performing chest compressions changes with the weight and gestational age of the newborn, which can be difficult to determine using visual approximation.

### SUMMARY

This Summary is provided to introduce a selection of concepts that are further described below in the Detailed Description. This Summary is not intended to identify key or essential features of the claimed subject matter, nor is it intended to be used as an aid in limiting the scope of the claimed subject matter.

In one aspect of the disclosure, an infant care system includes an infant support platform for supporting a neonate, at least one load cell configured to sense a force applied to the infant support platform, each load cell configured to receive at least a portion of an applied force on the infant support platform while the neonate is on the infant support platform and to generate a load signal indicative of the portion of the applied force received, and a control system. The control system is configured to receive the load signal over time during performance of a chest compression on the neonate and determine a slope value, an impulse value, a jerk value, or a combination of those values for the chest compression based on the load signal. The slope value is compared to a slope threshold, the impulse value is compared to an impulse threshold, the jerk value is compared to a jerk threshold to generate a comparison result for the chest compression. A care instruction is generated based on the comparison result.

In one embodiment, the control system is configured to identify the slope threshold, the impulse threshold, and/or the jerk threshold based on a gestational age of the neonate. Optionally, the gestational age is determined based on a weight of the neonate.

In another embodiment, the control system is configured to identify the slope threshold, the impulse threshold, and/or the jerk threshold based on a gestational age of the neonate obtained from or estimated based on a medical record of the mother or of the neonate.

In another embodiment, the control system is configured to identify the slope threshold, the impulse threshold, and/or the jerk threshold based on a weight of the neonate.

In another embodiment, the control system is further configured to determine a neonate weight for the neonate based on the load signal, and then identify the slope threshold, the impulse threshold, and/or the jerk threshold based on the neonate weight.

In another embodiment, the control system is further configured to determine a chest compression modification based on the comparison result, and then generate the care instruction by controlling a display device to generate a visual, audible, and/or haptic instruction based on the chest compression modification.

In another embodiment, the control system is further configured to determine an amplitude and/or a period of the chest compression and determine a chest compression modification based further on a difference between the amplitude and an amplitude threshold and/or a between the period and a period threshold.

In another embodiment, the control system is further configured to determine a chest compression rate based on the load signal during at least two consecutive chest compressions and determine a chest compression modification based further on a difference between the chest compression rate and a rate threshold.

In another embodiment, each of the slope value, the impulse value, and/or the jerk value is based on an increasing portion of the load signal associated with application of the chest compression.

In another embodiment, each of the slope value, the impulse value, and/or the jerk value is based on a decreasing portion of the load signal associated with release of the chest compression.

In another embodiment, the control system is further configured to determine a first slope value, a first impulse value, and/or a first jerk value based on an increasing portion of the load signal associated with application of the chest compression, and determine a second slope value, a second impulse value, and/or a second jerk value based on a decreasing portion of the load signal associated with release of the chest compression.

In another embodiment, the control system is further configured to compare a first slope value, a first impulse value, or a first jerk value to a first threshold to generate a first comparison result, and compare a second slope value, a second impulse value, or a second jerk value to a second threshold to generate a second comparison result, and then generate the care instruction based on the first comparison result and the second comparison result.

In another embodiment, the control system is further configured to determine a mattress resistance value and/or a mattress capacitance value, and identify the slope threshold, the impulse threshold, and/or the jerk threshold based on the mattress resistance value and/or the mattress capacitance value.

In another embodiment, the control system is further configured to generate a first comparison result for a first chest compression and generate a second comparison result for a second chest compression, and generate the care instruction based on the first comparison result and the second comparison result.

In another aspect of the disclosure, a method of monitoring a neonate includes sensing a force applied to an infant support platform supporting a neonate, each load cell configured to receive at least a portion of an applied force on the infant support platform while the neonate is on the infant support platform and to generate a load signal indicative of the portion of the applied force received. The load signal over time is analyzed during performance of a chest compression on the neonate, including determining a slope value, an impulse value, and/or a jerk value for the chest compression based on the load signal and comparing the slope value to a slope threshold, the impulse value to an impulse threshold, and/or the jerk value to a jerk threshold to generate a comparison result for the chest compression. A user interface device is controlled to generate a care instruction based on the comparison result.

In one embodiment, the method further includes determining a neonate weight for the neonate based on the load signal and then identifying the slope threshold, the impulse threshold, and/or the jerk threshold based on the neonate weight.

In another embodiment, the method further includes determining a chest compression modification based on the comparison result and generating the care instruction by controlling a display device to display a visual instruction and/or comparing an audio or haptic device to generate an audio or haptic care instruction based on the chest compression modification.

In another embodiment, the method further includes determining an amplitude and/or a period of the chest compression and determining a chest compression modification based further on a difference between the amplitude and an amplitude threshold and/or a between the period and a period threshold.

In another embodiment, the method further includes determining a chest compression rate based on the load signal during at least two consecutive chest compressions, and determining a chest compression modification based further on a difference between the chest compression rate and a rate threshold.

In another embodiment, each of the slope value, the impulse value, and/or the jerk value is based on an increasing portion of the load signal associated with application of the chest compression.

In another embodiment, each of the slope value, the impulse value, and/or the jerk value is based on a decreasing portion of the load signal associated with release of the chest compression.

In another embodiment, the method further includes determining a first slope value, a first impulse value, and/or a first jerk value based on an increasing portion of the load signal associated with application of the chest compression, and determining a second slope value, a second impulse value, and/or a second jerk value based on a decreasing portion of the load signal associated with release of the chest compression.

In another embodiment, the method further includes comparing a first slope value, a first impulse value, or a first jerk value to a first threshold to generate a first comparison result, and comparing a second slope value, a second impulse value, or a second jerk value to a second threshold to generate a second comparison result, and then generating the care instruction based on the first comparison result and the second comparison result.

In another embodiment, the method further includes determining a mattress resistance value and/or a mattress capacitance value, and identifying the slope threshold, the impulse threshold, and/or the jerk threshold based on the mattress resistance value and/or the mattress capacitance value.

In another embodiment, the method further includes generating a first comparison result for a first chest compression and generating a second comparison result for a second chest compression, and then generating the care instruction based on the first comparison result and the second comparison result.

In one aspect of the disclosure, an infant care system includes an infant support platform for supporting a neonate, at least one load cell configured to sense a force applied to the infant support platform, each load cell configured to receive at least a portion of an applied force on the infant support platform while the neonate is on the infant support platform and to generate a load signal indicative of the portion of the applied force received, and a control system. The control system is configured to receive the load signal over time during performance of a chest compression on the neonate and determine at least one morphological feature of the load signal and compare the morphological feature to a corresponding threshold. A user interface device is controlled to generate a care instruction based on the comparison result.

In one embodiment, the at least one morphological feature includes at least one of a slope value, an impulse value, and/or a jerk value for the chest compression. In a further embodiment, the at least one morphological feature further includes at least one of amplitude, a period, and/or a chest compression rate for at least one chest compression cycle.

In another embodiment, the at least one morphological feature includes a difference in area under a curve between the load signal and an ideal load signal for at least one chest compression cycle.

In another embodiment, the at least one morphological feature includes at least one of a maximum amplitude difference and a minimum amplitude difference between the load signal and an ideal load signal for at least one chest compression cycle.

In another aspect of the disclosure, a method of monitoring a neonate includes sensing a force applied to an infant support platform supporting a neonate, each load cell configured to receive at least a portion of an applied force on the infant support platform while the neonate is on the infant support platform and to generate a load signal indicative of the portion of the applied force received. The load signal over time is analyzed during performance of a chest compression on the neonate, including identifying at least one morphological feature of the load signal and comparing the morphological feature to a corresponding threshold. A user interface device is controlled to generate a care instruction based on the comparison result.

In one embodiment, the at least one morphological feature includes at least one of a slope value, an impulse value, and/or a jerk value for the chest compression. In a further embodiment, the at least one morphological feature further includes at least one of amplitude, a period, and/or a chest compression rate for at least one chest compression cycle.

In another embodiment, the at least one morphological feature includes a difference in area under a curve between the load signal and an ideal load signal for at least one chest compression cycle.

In another embodiment, the at least one morphological feature includes at least one of a maximum amplitude difference and a minimum amplitude difference between the load signal and an ideal load signal for at least one chest compression cycle.

Various other features, objects, and advantages of the invention will be made apparent from the following description taken together with the drawings.

### BRIEF DESCRIPTION OF THE DRAWINGS

The present disclosure is described with reference to the following Figures.
FIG. 1 is a schematic diagram of an infant care system in accordance with the present disclosure.
FIG. 2 depicts an exemplary control system associated with an infant care system.
FIG. 3 depicts an exemplary control system associated with an infant care system.
FIG. 4 is a graph depicting an exemplary method of operating an infant care system according to embodiments of the present disclosure.
FIG. 5 is a graph depicting an exemplary method of operating an infant care system according to embodiments of the present disclosure.
FIGS. 6-10 are flow charts depicting exemplary methods of operating an infant care system according to embodiments of the present disclosure.

### DETAILED DESCRIPTION

In the present description, certain terms have been used for brevity, clarity and understanding. No unnecessary limitations are to be inferred therefrom beyond the requirement of the prior art because such terms are used for descriptive purposes only and are intended to be broadly construed.

As used herein, unless otherwise limited or defined, discussion of particular directions is provided by example only, with regard to particular embodiments or relevant illustrations. For example, discussion of "top," "bottom," "front," "rear," "left," "right," "horizontal," "vertical," and "longitudinal" features and/or relative motion, e.g., movement "up" and "down," is generally intended as a description only of the orientation of such features relative to a reference frame of a particular example or illustration. Correspondingly, for example, a "top" feature may sometimes be disposed below a "bottom" feature (and so on), in some arrangements or embodiments. Additionally or alternatively, embodiments may be arranged in a different orientation such that "top" and "bottom" features are arranged horizontally relative to each other, for example in a "left-to-right" orientation.

The use herein of the terms "including," "comprising," or "having," and variations thereof, is meant to encompass the elements listed thereafter and equivalents thereof, as well as additional elements. Embodiments recited as "including," "comprising," or "having" certain elements are also contemplated as "consisting essentially of" and "consisting of" those certain elements.

Chest compressions may be performed on newborns after birth in labor and delivery and within specialized care areas such as neonatal intensive care. The inventors have recognized that chest compression technique and performance is inconsistent across clinicians and is often not optimized for a particular neonate and/or its care environment. Further, the inventors have recognized that chest compression optimization will change significantly based on the neonate's gestational age, and that optimal chest compression technique, including optimal force magnitude and rate of applied force, varies significantly across populations of neonates having different weights.

The inventors have recognized a need to provide caregivers with more accurate guidance on how to perform and optimize chest compressions for a particular neonate. Accordingly, the inventors have developed the disclosed system and a method for monitoring a neonate undergoing chest compressions. The system utilizes a load sensor under the neonate, such as integrated within the infant support platform, to detect the load applied to the infant care platform during performance of chest compressions. The load sensor is communicatively connected to a control system which operates to provide feedback to a caregiver performing the chest compressions based on the load measurement signal. The system is configured to determine a slope value, an acceleration value, and/or a jerk value for the chest compression based on the load signal and then to assess the chest compression by comparing one or more of those values to a threshold. In some embodiments, the system is configured to determine the threshold based on a gestational age of the neonate, which may be based on a weight of the neonate measured by the load sensor.

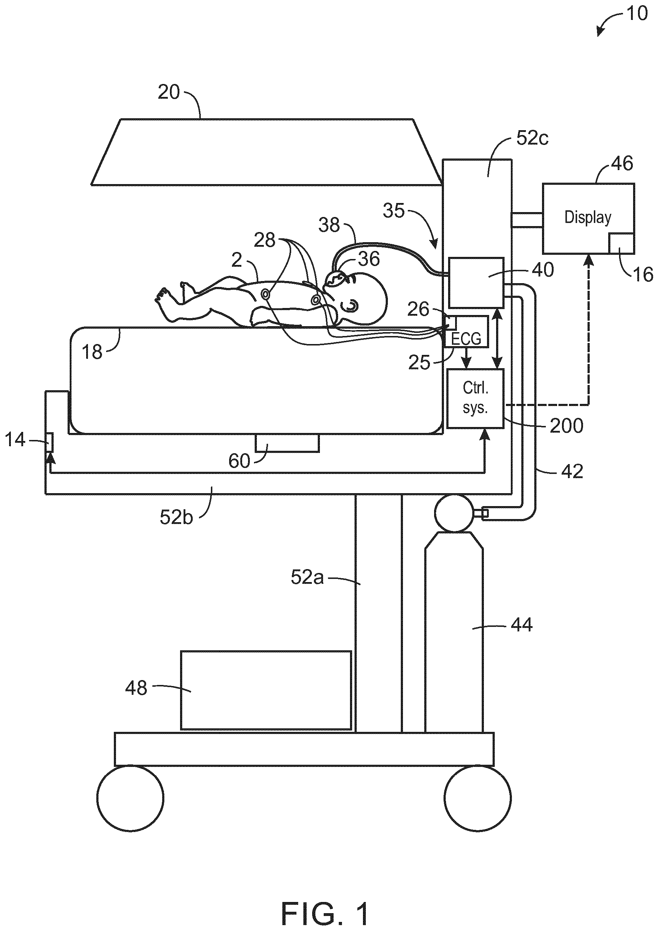

FIG. 1 depicts an exemplary infant care system 10. In the embodiment of FIG. 1 the exemplary infant care system 10 is a radiant warmer having a warmer system 20 that includes a radiant heater that directs infrared energy towards the infant 2 in order to provide a heated environment for the infant 2. In other embodiments the infant care system 10 may include an incubator system, alone or in combination with a radiant warmer, or may be another type of neonatal care device. In still other embodiments infant care system 10 may include just platform 18 supporting the infant 2 with no incubator or warmer elements. The infant care system 10 preferably provides easy and unimpeded access to the infant 2 for purposes of providing resuscitative care within the first few minutes after birth. The position of the infant 2 in FIG. 1 is not intended to be indicative or representative of actual or appropriate position of an infant for resuscitation. When performing resuscitative care to an infant in the first few minutes after birth, the infant 2 is often placed with their head proximal to the food side of platform 18. When performing resuscitative care to an infant in other neonatal environments, the infant 2 is often placed with their head distal to the food side of platform.

The exemplary infant care system 10 comprises a frame structure 52, which provides structural support for and houses the various aspects of the infant care system 10. The frame structure 52, for example, may include structural support elements supporting the weight of the system components, as well as exterior or casing elements that enclose and protect the system components and/or provide an attractive facade. In the example shown at FIG. 1, the frame structure 52 includes a base portion 52a (which may support and/or house various aspects of the system such as a battery 48 and/or a gas supply tank 44), a platform support structure 52b portion that supports the platform 18 on which the infant 2 lays, and a vertical panel structure 52c (which may support various user interface and control systems, for example). The schematic diagram of FIG. 1 is for purposes of exemplifying just one embodiment of the infant care system 10 of the present disclosure, and in other embodiments the system 10 may include any of various additional system elements and the system elements may be arranged on the frame structure 52 in various ways not exemplified in the schematic diagram provided in FIG. 1.

The infant care system 10 may include a battery 48 to power the various systems and devices thereon. The battery 48 may be positioned, for example, on the base structure 52a, such as at a location that is easily accessible to recharge or replace that battery 48. The charging status of the battery 48 may be monitored by a power control model which may be incorporated into the control system 200. Alternatively or additionally, the infant care system 20 may receive power from a grid system such as being plugged into an outlet connected to the AC power grid for a health care facility.

Devices and systems for monitoring and providing resuscitation and other therapies to the infant 2 are incorporated into the infant care system 10. In the depicted embodiment, the infant care system 10 includes an ECG monitor 25 housed on the frame structure 52. The ECG monitor 25 receives cardiac potentials from the electrodes 28 connected to the infant 2 at the right arm RA, left arm LA and left leg LL positions. In other embodiments, the ECG monitor may receive cardiac potentials from any number of two or more electrodes connected to the infant in any of various electrode arrangements. The ECG electrodes 28 are connected to the ECG monitor 25 via an ECG connection port 26 configured to receiver connectors of the two or more ECG electrodes 28. In various embodiments, the ECG connection port 26 may take any of various forms. The ECG electrode connection port provides a physical contact point 26*a* for connection with each electrode 28, which may be bundled together in a single connector that mates with and is separately received by the ECG connection port 26. In still other embodiments, the ECG electrodes 28 may be wireless electrodes, and in such an embodiment the ECG connection port 26 may comprise one or more wireless receiver transmitters in communication with two or more ECG wireless electrodes.

In some embodiments some or all of the physical circuitry and/or software comprising the ECG monitor 25 may be incorporated within the control system 200. In general, the control system 200 may comprise several separate computing systems, or control sub-systems in the ECG monitor 25, the ventilator device 40, and/or various other control sub-systems for controlling various aspects of the system 10, such as a display control sub-system, a speaker control sub-system, an auditory processing system associated with the microphone 14, etc. in various embodiments, all such sub-systems may be provided on separate hardware systems, or any or all of them may be combined together on in single set of hardware and software, and together may be generally referred to herein as the control system for the infant care system 10.

The infant care system 10 may further include devices and systems for providing ventilation support for the infant. In the schematic example of FIG. 1, a breathing circuit 35 for providing gas to the infant 2 includes a ventilator device 40, such as a continuous positive airway pressure (CPAP) device, a positive pressure ventilation (PPV) device, or a positive end expiratory pressure (PEEP) device (or a ventilator device providing all three respiratory therapies). In the embodiment, the ventilator device 40 receives a gas supply from the supply line 42 connected to gas supply tank 44 supported on the base structure 52*a*. the ventilator device 40 regulates the gas supply as appropriate to provide resuscitative and/or respiratory assistance to the infant 2. In the depicted scenario, the ventilator device 40 connects to a breathing tube 38 supplying gas to the infant through a mask 36 applied over the infant's nose and mouth. In other embodiments, gas may be delivered to the patient via another device, such as an endotracheal tube, a laryngeal mask, nasal cannula, or the like, and any such gas delivery system or element may connect to the breathing tube 38 in order to deliver gas from the gas supply 44 to the patient.

The care system 10 may include various user interfaces devices for controlling various aspects of the system. Such user interface devices may include a display device 46 controllable to provide physiological information about the infant 2 and/or the status of various aspects of the system. For example, the display 46 may be controllable by the control system 200 to display any physiological information measured from the patient. Additionally, the display 46 may be controlled to display information regarding the infant care system 20, ventilator device 40, or ECG monitor 25, such as the mode of operation or other pertinent information regarding those systems and devices. In certain embodiments, the display device 46 may be a touch screen capable of providing user control inputs through which a clinician can control the various systems and devices comprising the infant warming system 10.

The infant care system 10 may further include a microphone 14 configured to detect voice inputs from a clinician, such as during operation of the system in resuscitation mode as described herein. The system may further include a speaker 16, which may be incorporated into the display device 46 or elsewhere on the infant care system, that produces audible alerts, alarms, instructions, or the like to facilitate care of the infant.

The infant care system 10 includes at least one load cell 60 positioned below the infant and configured to measure a force applied to the infant on in the infant platform throughout the resuscitation period. In the depicted embodiment, the infant care system 10 includes a load cell 60 housed beneath platform 18. The load cell 60 is configured to sense a force being applied to platform 18, which may be the result of the weight of the infant 2 and may also be a result of a clinician performing chest compressions on the infant 2 positioned on the platform. While the example illustrates a single load cell 60, in other embodiments the system may include multiple load cells configured to measure a portion of the load distributed by the platform. Whether a single load cell 60 or multiple, the load cells 60, the load cells may be configured to capture the load fluctuations from performance of chest compressions on the neonate whether it is placed with its head proximal to or distal to the food side of platform.

Each load cell 60 is configured to generate a load signal indicative of the applied force received. The load cell 60 is communicatively connected to control system 200 and is configured to generate a load signal representing the load measurements over time. For example, the load cell may be configured to measure the load at a predetermined sample rate and to communicate a digital load signal representing the sampled load to the controller 200. For example, the sample rate may be 50Hz or 100Hz, and in various embodiments may be configured to be at least 10Hz.

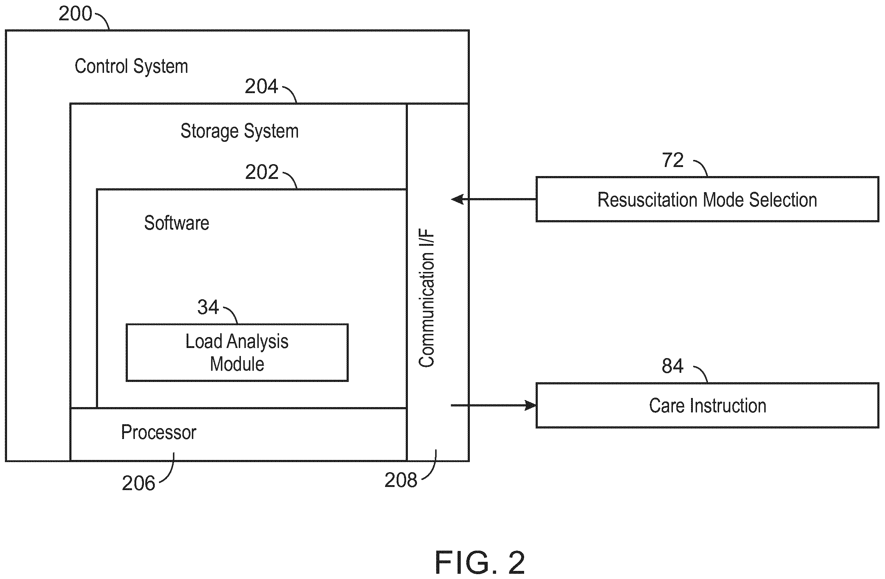

FIG. 2 provides a schematic diagram of an exemplary control system 200 associated with or comprised in the infant care system 10. In the depicted embodiment, the control system 200 includes software 202 stored in storage system 204 and executable by the processor 206 to perform the functions described herein. The storage system 202 may be implemented as a single storage device, or be distributed across multiple storage devices or sub-systems that cooperate to store computer readable instructions, data structures, program modules, or other data. The storage system 202 may include volatile and/or non-volatile systems, and may include removable and/or non-removable media implemented in any method or technology for storage of information. The storage media may include non-transitory and/or transitory storage media, including random access memory, read only memory, magnetic discs, optical discs, flash memory, virtual memory, and non-virtual memory, magnetic storage devices, or any other medium which can be used to store information and be accessed by an instruction execution system, for example. A communication interface 208 provides communication between the control system 200 and peripheral devices, such as the user interface devices.

In the depicted example, the software 202 includes a load analysis module 34 comprising instructions executable by a processor 206 to analyze the load signal(s) from the load sensor(s) 60 and to generate one or more care instructions 84 via a user interface device, such as the display 46 and/or the speaker 16, to assist a clinician in providing resuscitative care to the infant 2. As described further below, exemplary care instructions 84 could include a chest compression instruction such as an instruction to increase or decrease the force, impulse, and/or jerk applied during a chest compression. The load analysis module 34 may be activated in response to a user input, such as input of a resuscitation mode selection 72, and/or upon detection of a force applied to load cell 60, or load sensor, consistent with a force applied during a chest compression. Alternatively or additionally, the load analysis module 34 may be activated upon receipt of an input from a user indicating chest compressions are beginning.

The load analysis module 34 is configured to receive and analyze the load signal over time from the one or more load cell(s) 60. Where more the infant care system includes more than one load sensor positioned under the platform or under the neonate, the load analysis module 34 may be configured to select the load signal with the largest amplitude or to generate and analyze a total load signal. For example, the total load signal may be the sum of all of the load signals or the average of all of the load signals.

Load analysis module 34 is configured to determine a slope value of the magnitude of the load signal, or a portion of the load signal, representing the force applied over time. The slope value may be, for example, an average slope of a section of the load signal, such as an average slope of an increasing portion of the load signal between a minimum point and a maximum point representing the application of a chest compression on the neonate by a clinician. Alternatively, the average slope may be calculated between two points in the increasing portion of the signal, such as 5% and 95% of the total amplitude.

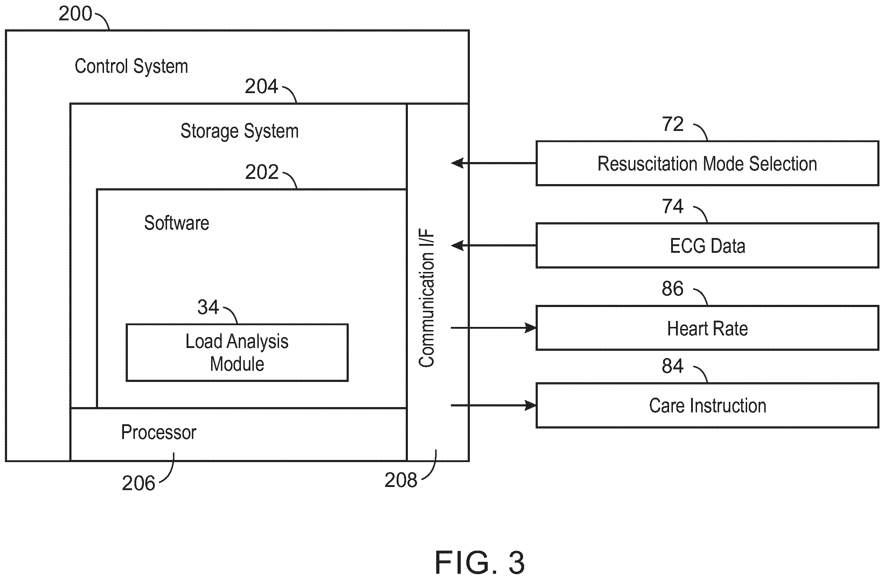

FIG. 3 provides a schematic diagram of an exemplary control system 200 associated with or comprised in the infant care system 10. In general the embodiment of FIG. 3 is most similar to that of FIG. 2 and is thus shown with like reference numbers for brevity. In the depicted embodiment, the control system 200 includes software 202 stored in storage system 204 and executable by the processor 206 to perform the functions described herein. In the depicted example, the software 202 includes a load analysis module 34 comprising instructions executable by a processor 206 to analyze the load signal(s) from the load sensor(s) 60 and to generate one or more care instructions 84 via a user interface device, such as the display 46 and/or the speaker 16, to assist a clinician in providing resuscitative care to the infant 2. As described further below, exemplary care instructions 84 could include a chest compression instruction such as an instruction to increase or decrease the force, impulse, and/or jerk applied during a chest compression. The load analysis module 34 may be activated in response to a user input, such as input of a resuscitation mode selection 72, and/or upon detection of a force applied to load cell 60, or load sensor, consistent with a force applied during a chest compression. Alternatively or additionally, the load analysis module 34 may be activated upon receipt of an input from a user indicating chest compressions are beginning.

The load analysis module 34 is configured to receive and analyze the load signal over time from the one or more load cell(s) 60. Where more the infant care system includes more than one load sensor positioned under the platform or under the neonate, the load analysis module 34 may be configured to select the load signal with the largest amplitude or to generate and analyze a total load signal. For example, the total load signal may be the sum of all of the load signals or the average of all of the load signals.

Load analysis module 34 is configured to determine a slope value of the magnitude of the load signal, or a portion of the load signal, representing the force applied over time. The slope value may be, for example, an average slope of a section of the load signal, such as an average slope of an increasing portion of the load signal between a minimum point and a maximum point representing the application of a chest compression on the neonate by a clinician. Alternatively, the average slope may be calculated between two points in the increasing portion of the signal, such as 5% and 95% of the total amplitude.

Control system 200 is further configured to receive ECG data 74 from ECG monitor 25 as described above. ECG data 74 is converted into a heart rate 86. Heart rate 86 is then output to ECG monitor 25 or display 46. A low heart rate may indicate a need for resuscitation to begin. Where the heart 86 is below a threshold heartrate control system 200 may generate an alert to inform caregivers that a need for resuscitation is indicated. Additionally, heart rate 86 may be used to further monitor chest compressions and indicate when resuscitation has been successful or needs to continue.

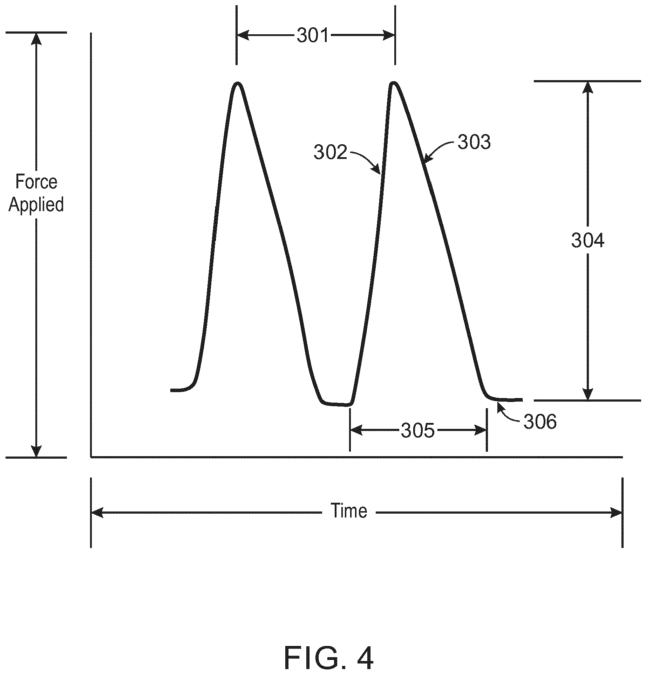

As illustrated in FIG. 4, the slope, impulse, and jerk values are calculated from the load signal, which is seen charted with respect to time. A chest compression cycle is defined by an increasing portion 302 of the load signal followed by a decreasing portion 303 of the load signal. The slope, impulse, and jerk values may be calculated for the increasing portion 302 of the load signal associated with the application of the chest compression and/or the decreasing portion 303 of the load signal associated with release of the chest compression.

The load analysis module 34 may be further configured to determine an impulse value, where the impulse is the derivative of the magnitude of force with respect to time (i.e., the load signal). The impulse value is derived from or based on the impulse and may be, for example, an average impulse of a section of the load signal, such as an average impulse of an increasing portion of the load signal between a minimum point and a maximum point representing the application of a chest compression on the neonate by a clinician.

Further, load analysis 34 may be configured to determine a jerk value, wherein jerk is the derivative of impulse with respect to time (or a second derivative of the load signal). The jerk value is derived from or based on the jerk and may be, for example, an average jerk of a section of the load signal, such as an average jerk of an increasing portion of the load signal between a minimum point and a maximum point representing the application of a chest compression on the neonate by a clinician.

Load analysis module 34 is further configured to compare the slope, impulse, and jerk values calculated by the load analysis module 34 to threshold slope, impulse, and jerk values which are stored in the storage system 204. The system may be configured to identify threshold slope, impulse, and jerk values to be utilized for a particular infant based on a gestational age of the infant 2. The gestational age is generally the duration of pregnancy, from the time of conception (or estimated conception) to the time that the resuscitation is being performed (which may be at the time of birth of the infant). Storage system 204 may contain different threshold values for different gestational ages, which may be selected by the control system based on input by a caregiver (e.g., caregiver input of the neonate's gestational age in days or weeks).

Alternatively or additionally, the load analysis module 34 may be configured to determine the estimated gestational age, or to otherwise select one or more thresholds, based on a weight of an infant 2. Alternatively, the gestational age may be user-inputted or may be determine an expected gestational age based on information in the mother's medical record. The system may be configured to determine the weight of the infant 2 by measuring the force applied to the infant support platform 18 before the commencement of chest compressions. Alternatively or additionally, load analysis module 34 may be configured to determine a weight of an infant 2 by measuring or estimating the minimum of the periodic force over time applied to infant support platform 18 during chest compressions. FIG. 4 shows minimum force 306, which may be used to estimate the weight of the infant. For example, where a weight of the infant is not or cannot be determined prior to the start of chest compressions, the weight of the infant may be determined as the lowest measured force by the load sensor over the course of the resuscitation period, or a portion thereof. The load analysis module 34 then accesses threshold values from storage system 204 based on the weight, the estimated gestational age, or the inputted gestational age. For example, the storage system may store a table comprising one or more stored threshold values-e.g., a slope threshold, an impulse threshold, and/or a jerk threshold-indexed based on gestational age, weight, or both.

Based on the comparison to the threshold slope, impulse, and jerk values the load analysis module 34 generates a care instruction 84. The care instruction 84 may be in auditory or visual form and is designed to provide feedback to the caregiver regarding the accuracy of their chest compression technique. The load analysis module 34 may be configured to determine a chest compression modification based on comparing the calculated slope, impulse, and jerk to the threshold values and the care instruction 84 may communicate that modification to the clinician. A chest compression modification may be an increase or decrease in the force applied to the infant 2 during chest compressions, an increase or decrease in the impulse applied during chest compressions, and/or an increase or decrease in jerk applied during chest compressions. Alternatively or additionally, a chest compression modification may be an instruction regarding compression depth or compression force and/or the rate at which it is applied-e.g., an instruction to press harder and apply the force faster, or to apply the same amount of force but faster between the start and finish of the compression, etc.

The control system 200 may be further configured to determine an amplitude 304 and a period 305 of the chest compression. As can be seen in FIG. 4, where the force applied to load cell 60 is charted over time, the amplitude 304 is equal to the maximum force applied minus the minimum force applied. The minimum force measured by the load cell over time may be considered as the neonate's weight. The amplitude 304 is the maximum value minus the minimum value, and may be considered equal to the maximum force applied by the clinician in the course of a given chest compression cycle. The period 305 is defined as the amount of time that elapses from detection of an increase in force from the minimum force applied value to the next detection of the minimum force applied value. This is equivalent to the time that elapses between the start of a chest compression cycle and the end of the chest compression cycle. An amplitude threshold and a period threshold may be stored in storage system 204. These thresholds may vary based on gestational age. Load analysis module 34 may be configured to compare the calculated amplitude 304 and period 305 to the threshold values and generate a care instruction 84 including instructing the caregiver to increase or decrease the amplitude or period of the chest compression.

The control system 200 may further be configured to determine a rate 301. As can be seen in FIG. 4, a rate 301 is defined as the time elapsed from detection of a maximum force applied to detection of the next maximum force applied. A rate threshold may be stored in storage system 204. The rate threshold may vary based on gestational age, which may be stored and accessed by the control system as described above. Load analysis module 34 may compare the calculated rate 31 with the threshold value and care instruction 84 may include instructing the caregiver to increase or decrease the rate of chest compressions.

A second slope, second impulse, and/or second jerk value may be calculated based on the relaxation phase of the chest compression cycle. The load analysis module 34 may be further configured to compare the calculated second slope, second impulse, and second jerk values, with a second slope threshold, second impulse threshold, and second jerk threshold. The slope, impulse, and jerk value calculations may be determined based on the load signal, or a portion thereof, as described above. Control system 200 may be configured to adjust care instruction 84 to instruct the caregiver to increase or decrease the second slope, second impulse, and/or the second jerk.

Control system 200 may also be configured to determine a mattress resistance value and/or a mattress capacitance value based on the second impulse value. The signature of the load sensor signal, both rise and fall, or attack and decay, may be analyzed for impedance (resistance and capacitance). For example, a pattern recognition type of analysis may be utilized to show the various values of capacitance, which will be indicative of the mattress properties.

In some embodiments, control system 200 may be configured to generate a first comparison result for a first chest compression, including a comparison of a slope, impulse, and/or jerk with a threshold slope, impulse, and/or jerk and a second comparison result of the same values for a second chest compression. Care instruction 84 would then be based on both the first comparison result and the second comparison result.

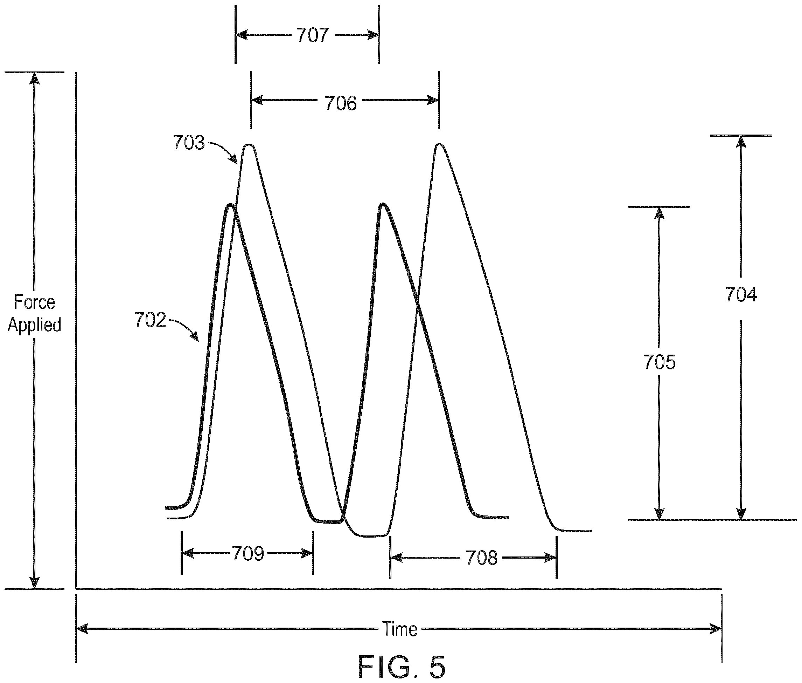

Referring to FIG. 5, an ideal load signal 702 is shown compared with a sensed curve 703. Sensed curve 703 represents the force applied to load cell 60 charted over time. Ideal curve 702 represents the ideal application of force over time for a neonate of a given gestational age. Control system 200 will compare ideal load signal 702, or curve, to the sensed signal 703 in order to generate care instructions. For example, the sensed amplitude 704 is compared to the ideal amplitude 705. Where, as in the example, the sensed amplitude 704 is greater than the ideal amplitude 705 control system 200 generates a care instruction to guide the caregiver to apply less force to the neonate. If the sensed amplitude 704 is less than the ideal amplitude 705 the control system 200 generates a care instruction to instruct the caregiver to apply more force during the compression cycle.

Further, where the calculated rate 706 is greater than the ideal rate 707 the control system 200 generates a care instruction instructing the caregiver to decrease the amount of time between the maximum force applied of consecutive compressions. If the calculated rate 706 is less than the ideal rate 707 control system 200 generates a care instruction to instruct the caregiver to increase the amount of time between the maximum force applied of consecutive compressions. Further, where sensed period 708 is greater than ideal period 709 control system 200 generates a care instruction to instruct the caregiver to decrease the amount of time force is applied during a chest compression. Alternatively, where sensed period 708 is less than ideal period 709 control system 200 generates a care instruction to instruct the caregiver to increase the amount of time force is applied during a chest compression.

Control system 200 is further configured to compare a slope of ideal load signal 702, calculated as described above, to a slope of sensed load signal 703. If the slope of ideal load signal 702 is greater than the slope of sensed load signal 703 control system 200 will generate a care instruction to instruct the caregiver to increase the rate at which force is applied during a chest compression. If the slope of ideal load signal 702 is less than the slope of sensed load curve 703 control system 200 will generate a care instruction to instruct the caregiver to decrease the rate at which force is applied during a chest compression.

Control system 200 is further configured to compare an impulse value of ideal load signal 702 with an impulse value of sensed load signal 703, calculated as described above. If the impulse value of the ideal load signal 702 is greater than the impulse value of the sensed load signal 703 control system 200 will generate a care instruction to instruct the caregiver to increase the impulse of applied force. If the impulse value of the ideal load signal 702 is less than the impulse value of the sensed load signal 703 control system 200 will generate a care instruction to instruct the caregiver to decrease the impulse of applied force.

Control system 200 is further configured to compare a jerk value of ideal load signal 702 with a jerk value of sensed load signal 703, calculated as described above. If the jerk value of the ideal load signal 702 is greater than the jerk value of the sensed load curve 703 control system 200 will generate a care instruction to instruct the caregiver to increase the jerk of applied force. If the jerk value of the ideal load signal 702 is less than the jerk value of the sensed load signal 703 control system will generate a care instruction to instruct the caregiver to decrease the jerk of applied force.

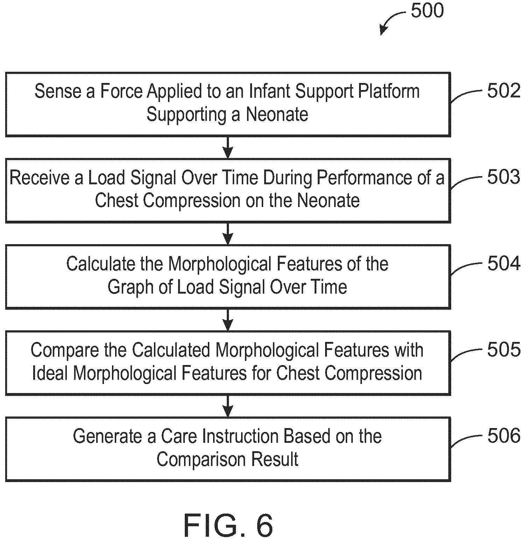

Referring to FIG. 6, a flow chart is shown describing a method 500 for monitoring a neonate undergoing chest compressions in accordance with embodiments of this disclosure. At 502 a force is sensed being applied to an infant support platform 18 supporting a neonate 2. At 503 a load signal is received corresponding to the load over time during a chest compression cycle performed on the neonate. At 504 the morphological features of the load signal over time are studied. These features may include slope, impulse, jerk, period, rate, amplitude, or any relevant morphological features. At 505 the morphological features of the sensed load signal over time are compared to ideal morphological features for a correctly applied chest compression. These ideal features may vary based on the neonate's weight and/or gestational age, such as age/weight based thresholds as described above. Alternatively or additionally, the system may be configured to compare a portion of the load signal to an ideal morphology, such as an ideal morphology selected based on the neonate's weight and/or gestational age. For example, the threshold comparison may result in a difference between the load signal morphology (e.g., over the compression period or a portion thereof) and the ideal morphology, such as a difference in area under the curve or some other metric of the difference. Finally, at step 506, a care instruction is generated based on the comparison between the measured morphological features and the ideal morphological features. The care instruction may include instructions to increase or decrease a slope, impulse, jerk, amplitude, rate, period, or other relevant morphological feature.

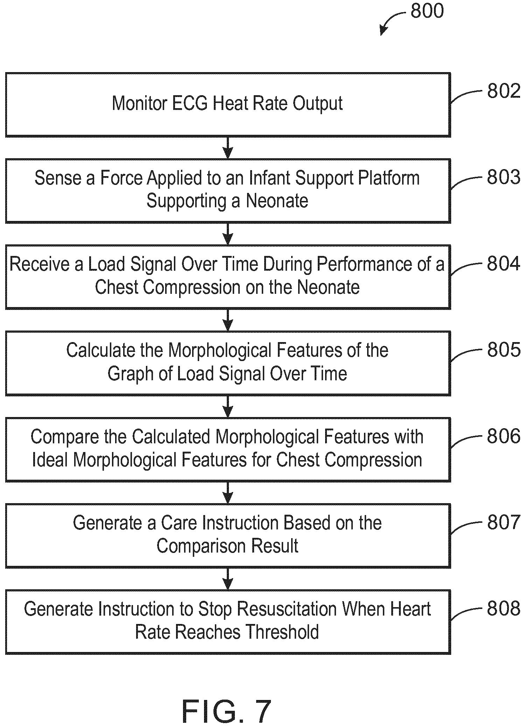

Referring to FIG. 7, a flow chart is shown describing a method 800 for monitoring a neonate undergoing chest compressions in accordance with embodiments of this disclosure. At 802 a neonate's heart rate is monitored by means of an ECG, as described above. When the neonate's heart rate drops below a safe heart rate threshold a care instruction is generated to alert caregivers that a need for resuscitation is indicated. At 803 a force is sensed being applied to an infant support platform 18 supporting a neonate 2. At 804 a load signal is received corresponding to the load over time during a chest compression cycle performed on the neonate. At 805 the morphological features of the load signal over time are studied. These features may include slope, impulse, jerk, period, rate, amplitude, or any relevant morphological features. At 806 the morphological features of the sensed load signal over time are compared to ideal morphological features for a correctly applied chest compression. These ideal features may vary based on the neonate's weight and/or gestational age, such as age/weight based thresholds as described above. Alternatively or additionally, the system may be configured to compare a portion of the load signal to an ideal morphology, such as an ideal morphology selected based on the neonate's weight and/or gestational age. For example, the threshold comparison may result in a difference between the load signal morphology (e.g., over the compression period or a portion thereof) and the ideal morphology, such as a difference in area under the curve or some other metric of the difference. At step 807, a care instruction is generated based on the comparison between the measured morphological features and the ideal morphological features. The care instruction may include instructions to increase or decrease a slope, impulse, jerk, amplitude, rate, period, or other relevant morphological feature. Finally, at step 808, the monitored heart rate is compared to a threshold safe heart rate. Once the monitored heart rate exceeds the threshold heart rate a care instruction is generated to instruct the caregiver to cease compressions as resuscitation has been successful.

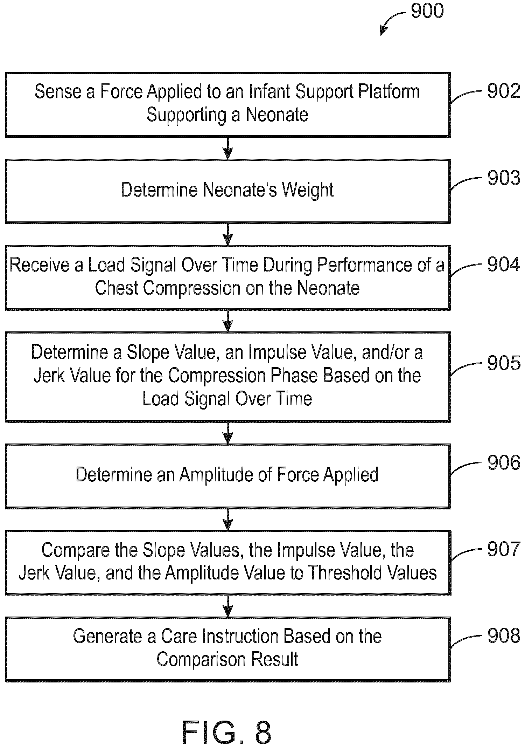

Referring to FIG. 8, a flow chart is shown describing a method 900 for monitoring a neonate undergoing chest compressions in accordance with embodiments of this disclosure. At 902 a force is sensed applied to an infant support platform supporting a neonate. At 903 a weight is determined for a neonate as described above. At 904 a load signal during performance of a chest compression is received and charted over time. At 905 the slope, impulse, and jerk values are calculated for the compression phase of the compression cycle, as described above. At 906 the amplitude of the chest compression is calculated. At 907 the slope, impulse, jerk, and amplitude values are compared to threshold values. These threshold values may vary based on a neonate's gestational age, which may be estimated utilizing a neonate's weight, as described above. Finally, at 908, a care instruction is generated based on the comparison of the measured features with the threshold values. The care instruction may include an instruction to increase or decrease the slope, impulse, jerk, or amplitude of chest compressions.

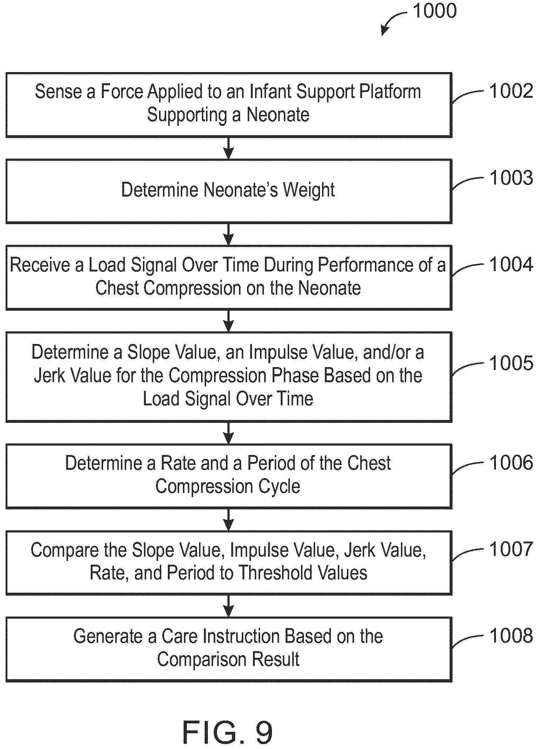

Referring to FIG. 9, a flow chart is shown describing a method 1000 a for monitoring a neonate undergoing chest compressions in accordance with embodiments of this disclosure. At 1002 a force is sensed applied to an infant support platform supporting a neonate. At 1003 a weight is determined for a neonate as described above. At 1004 a load signal during performance of a chest compression is received and charted over time. At 1005 the slope, impulse, and jerk values are calculated for the compression phase of the compression cycle, as described above. At 1006 the rate and period of the chest compression are calculated. At 1007 the slope, impulse, jerk, rate, and period are compared to threshold values. These threshold values may vary based on a neonate's gestational age, which may be estimated using a neonate's weight, as described above. Finally, at 908, a care instruction is generated based on the comparison of the measured features with the threshold values. The care instruction may include an instruction to increase or decrease the slope, impulse, jerk, rate, or period of chest compressions.

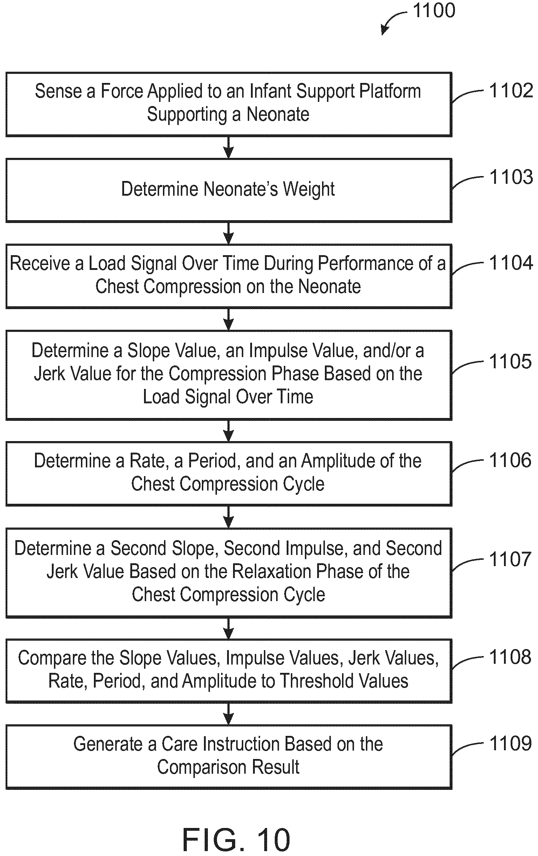

Referring to FIG. 10, a flow chart is shown describing a method 1100 for monitoring a neonate undergoing chest compressions in accordance with embodiments of this disclosure. At 1102 a force is sensed applied to an infant support platform supporting a neonate. At 1103 a weight is determined for a neonate, as described above. At 1104 a load signal during performance of chest compressions is received and charted over time. At 1105 the slope, impulse, and jerk values are calculated for the compression phase of the compression cycle, as described above. At 1106 the rate, period, and amplitude of the compression cycle are calculated as described above. At 1107 a second rate, second impulse, and second jerk are calculated based on the relaxation phase of the chest compression cycle, as described above. At 1108 the first and second slope values, first and second impulse values, first and second jerk values, rate, period, and amplitude are compared to threshold values. These threshold values may vary based on a neonate's gestational age, which may be estimated utilizing the neonate's measured weight, as described above. Finally, at 1109, a care instruction is generated based on the comparison of the measured features with the threshold values. The care instruction may include an instruction to increase or decrease a first or second slope, first or second impulse, first or second jerk, a period, or a rate.

This written description uses examples to disclose the invention, including the best mode, and also to enable any person skilled in the art to make and use the invention. Certain terms have been used for brevity, clarity, and understanding. No unnecessary limitations are to be inferred therefrom beyond the requirement of the prior art because such terms are used for descriptive purposes only are intended to be broadly construed. The patentable scope of the invention is defined by the claims, and may include other examples that occur to those skilled in the art. Such other examples are intended to be within the scope of the claims if they have features or structural elements that do not differ from the literal language of the claims, or if they include equivalent features or structural elements with insubstantial differences from the literal language of the claims.

## Claims

1. An infant care system comprising:
an infant support platform for supporting a neonate;
at least one load cell configured to sense a force applied to the infant support platform, each load cell configured to receive at least a portion of an applied force on the infant support platform while the neonate is on the infant support platform and to generate a load signal indicative of the portion of the applied force received; and
a control system configured to:
receive the load signal over time during performance of a chest compression on the neonate;
determine a slope value, an impulse value, a jerk value, or a combination thereof for the chest compression based on the load signal;
compare the slope value to a slope threshold, the impulse value to an impulse threshold, or the jerk value to a jerk threshold, or a combination to generate a comparison result for the chest compression; and
generate a care instruction based on the comparison result.

2. The system of claim 1, wherein the control system is configured to identify the slope threshold, the impulse threshold, or the jerk threshold based on a gestational age of the neonate.

3. The system of claim 2, wherein the gestational age is based on a weight of the neonate.

4. The system of claim 1, wherein the control system is further configured to:
determine a neonate weight for the neonate based on the load signal; and
identify the slope threshold, the impulse threshold, or the jerk threshold based on the neonate weight.

5. The system of claim 1, wherein the control system is further configured to:
determine a chest compression modification based on the comparison result; and
generate the care instruction by controlling a display device to generate a visual or audible instruction based on the chest compression modification.

6. The system of claim 5, wherein the control system is further configured to:
determine at least one of an amplitude and a period of the chest compression; and
determine the chest compression modification based further on a difference between the amplitude and an amplitude threshold or a between the period and a period threshold.

7. The system of claim 5, wherein the control system is further configured to:
determine a chest compression rate based on the load signal during at least two consecutive chest compressions; and
determine the chest compression modification based further on a difference between the chest compression rate and a rate threshold.

8. The system of claim 1, wherein each of the slope value, the impulse value, or the jerk value is based on an increasing portion of the load signal associated with application of the chest compression.

9. The system of claim 1, wherein the control system is further configured to:
determine a first slope value, a first impulse value, or a first jerk value based on an increasing portion of the load signal associated with application of the chest compression; and
determine a second slope value, a second impulse value, or a second jerk value based on a decreasing portion of the load signal associated with release of the chest compression.

10. The system of claim 9, wherein the control system is further configured to:
compare the first slope value, the first impulse value, or the first jerk value to a first threshold to generate a first comparison result;
compare the second slope value, the second impulse value, or the second jerk value to a second threshold to generate a second comparison result; and
generate the care instruction based on the first comparison result and the second comparison result.

11. The system of claim 9, wherein the control system is further configured to:
determine a mattress resistance value based on the second slope value or a mattress capacitance value based on the second impulse value; and
identify the slope threshold, the impulse threshold, or the jerk threshold based on the mattress resistance value or the mattress capacitance value.

12. The system of claim 1, where the control system is further configured to:
generate a first comparison result for a first chest compression and generate a second comparison result for a second chest compression; and
generate the care instruction based on the first comparison result and the second comparison result.

13. A method of monitoring a neonate, the method comprising:
sensing a force applied to an infant support platform supporting the neonate, each load cell configured to receive at least a portion of an applied force on the infant support platform while the neonate is on the infant support platform and to generate a load signal indicative of the portion of the applied force received;
receiving the load signal over time during performance of a chest compression on the neonate;
determining a slope value, an impulse value, or a jerk value for the chest compression based on the load signal;
comparing the slope value to a slope threshold, the impulse value to an impulse threshold, or the jerk value to a jerk threshold to generate a comparison result for the chest compression; and
controlling a user interface device to generate a care instruction based on the comparison result.

14. The method of claim 13, further comprising:
determining a chest compression modification based on the comparison result;
generating the care instruction by controlling a display device to display a visual instruction based on the chest compression modification;
determining at least one of an amplitude and a period of the chest compression; or
determining the chest compression modification based further on a difference between the amplitude and an amplitude threshold or a between the period and a period threshold.

15. The method of claim 13, further comprising:
determining a first slope value, a first impulse value, a first jerk value, or a combination thereof based on an increasing portion of the load signal associated with application of the chest compression;
comparing the first slope value, the first impulse value, or the first jerk value to a first threshold to generate a first comparison result;
determining a second slope value, a second impulse value, a second jerk value, or a combination thereof based on a decreasing portion of the load signal associated with release of the chest compression;
comparing the second slope value, the second impulse value, or the second jerk value to a second threshold to generate a second comparison result; or
generating the care instruction based on the first comparison result and the second comparison result.
